# EUROPÄISCHE PATENTSCHRIFT

(11) **EP 2 395 917 B1**
(45) Veröffentlichungstag und Bekanntmachung des Hinweises auf die Patenterteilung: **31.10.2012**
(21) Anmeldenummer: 10702250.1
(22) Anmeldetag: 27.01.2010
(51) Int. Cl.: A61B 5/151

(54) **LANZETTE MIT TESTBEREICH**
LANCET COMPRISING A TEST REGION
LANCETTE COMPRENANT UNE ZONE DE TEST

(30) Priorität: 12.02.2009 EP 09001937
(43) Veröffentlichungstag der Anmeldung: 21.12.2011
(73) Patentinhaber: Roche Diagnostics GmbH, 68305 Mannheim (DE); F. Hoffmann-La Roche AG, 4070 Basel (CH)
(72) Erfinder: KONYA, Ahmet, D-67065 Ludwigshaven (DE); HÖRAUF, Christian, D-68723 Oftersheim (DE)
(74) Vertreter: Twelmeier Mommer & Partner
(86) Internationale Anmeldenummer: PCT/EP2010/000460
(87) Internationale Veröffentlichungsnummer: WO 2010/091785

(56) Entgegenhaltungen:
- EP-A- 1 961 381
- WO-A-2005/084530
- US-A1- 2006 293 611
- US-A1- 2008 082 023

## Beschreibung

Die Erfindung geht aus von einer Lanzette wie sie aus der EP 1 360 933 B1 oder der WO 2005/084530 A2 bekannt ist. Eine solche Lanzette hat einen Grundkörper mit einem Testbereich zur Untersuchung einer Körperflüssigkeitsprobe, ein von dem Grundkörper ausgehendes Stechelement zum Einstich in die Haut eines Patienten, das sich in einer Stichrichtung erstreckt, und eine Kapillarstruktur, um eine Körperflüssigkeitsprobe von dem Stechelement zu dem Testbereich zu transportieren. Die Erfindung betrifft ferner ein Stechsystem umfassend ein Stechgerät und derartige Lanzetten.

Eine lanzette mit den im Oberbegriff des Anspruchs 1 angegebenen Merkmalen ist aus der US 2006/0293611 bekannt.

Lanzetten, die mit dazu passenden Handgeräten ein Stechsystem bilden, werden beispielsweise von Diabetikern benötigt, die ihren Blutzuckerspiegel mehrmals täglich überprüfen müssen und dafür eine durch eine Stichwunde gewonnene Körperflüssigkeitsprobe benötigen. Ständiges Ziel bei der Entwicklung derartiger Stechsysteme ist es, diese so klein und kompakt wie möglich auszubilden. Dies gilt nicht nur für Handgeräte zur Messung einer Analytkonzentration, beispielsweise der Glukosekonzentration oder der Laktatkonzentration, sondern insbesondere auch für die von Handgeräten benötigten Lanzetten. Je kleiner die Lanzetten nämlich sind, desto grö-βer ist der Lanzettenvorrat, der in einem Handgerät mitgeführt werden kann.

Aufgabe der Erfindung ist es deshalb, einen Weg aufzuzeigen, wie ein kompaktes Stechsystem zur Untersuchung einer Körperflüssigkeitsprobe verwirklicht werden kann.

Diese Aufgabe wird durch eine Lanzette mit den im Anspruch 1 angegebenen Merkmalen gelöst. Vorteilhafte Weiterbildungen der Erfindung sind Gegenstand von Unteransprüchen. Die Erfindung wird ferner durch ein Stechsystem mit erfindungsgemäßen Lanzetten und einem Stechgerät gemäß Anspruch 10 gelöst.

In einer Draufsicht auf die Oberseite gesehen sind bei einer erfindungsgemäßen Lanzette das Stechelement und der Testbereich zueinander seitlich versetzt angeordnet und die Kapillarstruktur weist einen Abschnitt auf, der Flüssigkeit seitwärts transportiert.

Um Flüssigkeit seitwärts zu transportieren, weist die Kapillarstruktur beispielsweise einen Abschnitt auf, der in der Draufsicht gesehen quer, also beispielsweise senkrecht oder schräg, zur Längsrichtung des Stechelements verläuft. Der Betreffende Abschnitt kann geradlinig verlaufen, muss es aber nicht. Möglich ist es deshalb auch, dass dieser Abschnitt der Kapillarstruktur einer Draufsicht in einem Bogen verläuft.

Allgemein gesagt kann die Kapillarstruktur einen Abschnitt aufweisen, der einen Flüssigkeitstransport in einer Richtung bewirkt, die in einer Draufsicht auf die Oberseite des Grundkörpers eine senkrecht zur Längsrichtung des Stechelements verlaufende Komponente hat. Die Richtung kann gegebenenfalls weitere Komponenten haben, die Längsrichtung des Stechelements oder senkrecht zur Oberseite verlaufen.

Die erfindungsgemäße Anordnung der Kapillarstruktur ermöglicht es, den Testbereich in Bezug auf die Längsrichtung des Stechelements seitlich versetzt von dem Stechelement anzuordnen, so dass der Testbereich nicht in direkter Linie hinter dem Stechelement sitzt. Bei einer erfindungsgemäßen Lanzette sich deshalb erreichen, dass auch bei einer sehr kleinen Lanzette eine Ankopplung an einen Stechantrieb eines Stechgeräts möglich ist, ohne die Untersuchung einer Körperflüssigkeitsprobe in dem Testbereich zu beieinträchtigen.

Bei einer erfindungsgemäßen Lanzette ist ein Kopplungsbereich, an dem der Stechantrieb eines Stechgeräts ankoppelt, in gerader Linie hinter dem Stechelement und neben dem Testbereich angeordnet . Auf diese Weise kann die Länge der Lanzette ohne Einschränkung ihrer Funktionalität wesentlich verkürzt werden.

Insbesondere kann bei dem erfindungsgemäßen Stechsystem der Stechantrieb eines Stechgeräts in Stichrichtung hinter dem Stechelement an die Lanzette ankoppeln - was Kippmomente und deshalb auch schmerzerzeugende Vibrationen minimiert - und eine geradlinige Stichbewegung bewirken. Indem der Testbereich seitlich neben dem Kopplungsbereich angeordnet ist, bleibt der Testbereich dabei für eine Messeinheit zugänglich. Vorteilhaft kann an dem Testbereich einer erfindungsgemäßen Lanzette eine Messung vorgenommen werden, während die Lanzette an den Stechantrieb angekoppelt ist. Nach einem Stich ist deshalb bei einer erfindungsgemäßen Lanzette keine aufwendige Repositionierung in Bezug auf eine Messeinheit erforderlich. Das Handgerät eines erfindungsgemäßen Stechsystems kann deshalb trotz kleiner Lanzetten und kleiner Probenmengen mit einer relativ einfachen und kostengünstigen Mechanik auskommen.

Eine vorteilhafte Weiterbildung der Erfindung sieht vor, dass die Kapillarstruktur als Kapillarkanal ausgebildet ist, beispielsweise als eine Rinne oder ein Schlitz. Eine solche Rinne oder ein Schlitz können offen sein oder durch eine Abdeckung, beispielsweise eine Folie, bedeckt sein. Bevorzugt hat die Kapillarstruktur eine hydrophile Oberfläche, was sich insbesondere durch eine hydrophile Beschichtung, beispielsweise mit Heparin, erreichen lässt. Unter einer Kapillarstruktur ist dabei eine Struktur zu verstehen, die durch Kapillarkräfte einen Flüssigkeitstransport bewirkt.

Erfindungsgemäβ hat der Kapillarkanal einen Knick oder eine Kurve, wobei der Kapillarkanal im Bereich des Knicks oder der Kurve eine Querschnittsfläche aufweist, die höchstens so groß wie in einem angrenzenden Abschnitt ist. Indem in einem Knick die Ecken angerundet werden, lässt sich eine Zunahme des Abstands zwischen gegenüberliegenden Rändern des Kanals im Bereich des Knicks verhindern und somit für eine gleich bleibende Querschnittsfläche sorgen. Die Querschnittsfläche ist dabei jeweils senkrecht zur lokalen Strömungsrichtung zu orientieren.

Eine weitere vorteilhafte Weiterbildung der Erfindung sieht vor, dass Kapillarstruktur einen Flüssigkeitstransport in einer Ebene bewirkt. Geeignet Kapillarstrukturen lassen sich mit vorteilhaft geringem Aufwand ausbilden, insbesondere auf einer ebenen Oberseite des Grundkörpers, beispielsweise durch Ätzen.

Der Grundkörper einer erfindungsgemäßen Lanzette kann einstückig mit dem Stechelement ausgebildet sein, beispielsweise indem die Kontur des Grundkörpers mit dem von ihm ausgehenden Stechelement aus einem Blech herausgeschnitten wird, beispielsweise durch Laserstrahlschneiden, Stanzen oder Ätzen. Möglich ist es aber auch, dass der Grundkörper und das Stechelement separate Bauteile sind, die bei der Herstellung der Lanzette zusammengefügt werden.

Eine weitere vorteilhafte Weiterbildung der Erfindung sieht vor, dass der Testbereich in Bezug auf die Längsrichtung des Stechelements seitlich mindestens um die Breite des Stechelements versetzt angeordnet ist. Der Versatz ist dabei von der Mitte des Stechelements bis zur Mitte des Testbereichs zu messen. Das Stechelement hat dabei bevorzugt in einem an seine Spitze anschließenden Bereich, der bei einem Stich in die Haut eines Patienten eindringt, eine gleich bleibende Breite, kann sich jedoch bis zu dem Grundkörper auch zu nehmend verbreitern. Maßgeblich ist Breite des Stechelements am Ende des bei einem Stich bestimmungsgemäß in die Haut eines Patienten eindringenden Bereichs.

Eine weitere vorteilhafte Weiterbildung der Erfindung sieht vor, dass das Stechelement in einer Draufsicht außermittig an dem Grundkörper angeordnet ist, also von dem linken und dem rechten Rand des Grundkörpers unterschiedlich weit entfernt ist. Auf diese Weise kann eine besonders kompakte Lanzette mit einem seitlich versetzt von dem Stechelement angeordneten Testbereich geschaffen werden. Der Grundkörper kann sich seitlich von dem Stechelement mit einem den Testbereich tragenden Abschnitt weiter als auf der entgegen gesetzten Seite von dem Stechelement erstrecken, beispielsweise um die Hälfte weiter, doppelt so weit oder noch weiter.

Eine weitere vorteilhafte Weiterbildung der Erfindung sieht vor, dass der Testbereich als ein aufgeklebtes Testfeld mit Nachweisreagenzien ausgebildet ist. Entsprechende Testfelder mit Nachweisreagenzien zur fotometrischen oder elektrochemischen Bestimmung einer Analytkonzentration sind bei handelsüblichen Teststreifen, beispielsweise zur Bestimmung der Glukosekonzentration vorhanden und bedürfen deshalb keiner weiteren Erläuterung. Anstelle eines aufgeklebten Testfeldes kann der Testbereich beispielsweise auch als eine Küvette ausgebildet sein, in der eine Körperflüssigkeitsprobe optisch untersucht werden kann, insbesondere reagenzfrei untersucht werden kann.

Weitere Einzelheiten und Vorteile der Erfindung werden an Ausführungsbeispielen unter Bezugnahme auf die beigefügten Zeichnungen erläutert. Gleiche und einander entsprechende Teile sind dabei mit übereinstimmenden Bezugszahlen bezeichnet. Es zeigen:
- Fig. 1: eine schematische Darstellung eines Ausführungsbeispiels einer erfindungsgemäßen Lanzette in einer Draufsicht auf eine Oberseite ihres Grundkörpers;
- Fig. 2: eine schematische Darstellung eines weiteren Ausführungsbeispiels einer erfindungsgemäßen Lanzette;
- Fig. 3: eine schematische Darstellung eines weiteren Ausführungsbeispiels einer erfindungsgemäßen Lanzette;
- Fig. 4: eine schematische Darstellung eines Lanzettenträgerbands mit mehreren Lanzetten gemäß Figur 1; und
- Fig. 5: eine schematische Darstellung eines Ausführungsbeispiels eines Stechsystems.

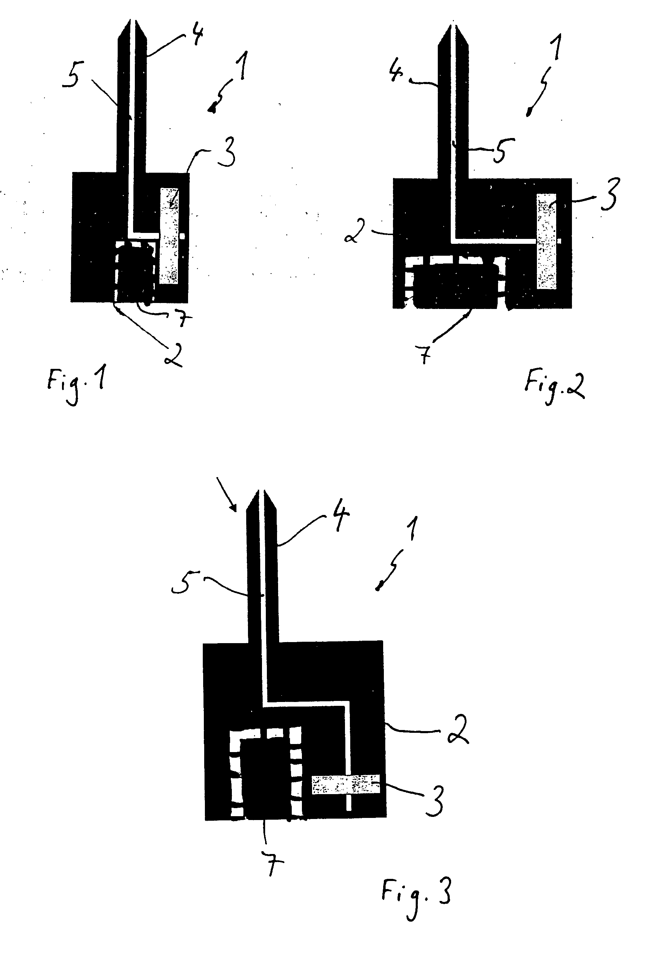

Die in Figur 1 dargestellte Lanzette 1 hat einen Grundkörper 2 mit einem Testbereich 3 zur Untersuchung einer Körperflüssigkeitsprobe, ein von dem Grundkörper 2 ausgehendes Stechelement 4 zum Einstich in die Haut eines Patienten und eine Kapillarstruktur 5, um eine Körperflüssigkeitsprobe von dem Stechelement 4 zu dem Testbereich 3 zu transportieren. Das Stechelement 4 erstreckt sich in einer Längsrichtung, die mit der Stichrichtung übereinstimmt, in der die Lanzette 1 bei Gebrauch eine geradlinige Stichbewegung ausführt.

Das Stechelement 4 ist in Stichrichtung gesehen seitlich versetzt von dem Testbereich 3 angeordnet. Die Kapillarstruktur 5 hat deshalb einen Abschnitt, der einen Flüssigkeitstransport quer zur Stichrichtung bewirkt. Bei dem dargestellten Ausführungsbeispiel ist der quer zur Längsrichtung des Stechelements 4 verlaufende Abschnitt der Kapillarstruktur 5 senkrecht zur Längsrichtung des Stechelements 4 angeordnet. Prinzipiell kann der quer zur Längsrichtung des Stechelements 4 verlaufende Abschnitt der Kapillarstruktur 5 aber auch schräg zur Längsrichtung des Stechelements 4 oder bogenförmig verlaufen.

Der quer zur Stichrichtung verlaufende Abschnitt der Kapillarstruktur 5 ist ebenso wie der Testbereich 3 auf dem Grundkörper 2 angeordnet. Die Kapillarstruktur 5 ist bevorzugt als ein Kapillarkanal ausgebildet, beispielsweise in Form einer Rinne oder eines durchgehenden Schlitzes. Die Kapillarstruktur 5 erstreckt von einem vorderen Bereich des Stechelementes, der bei einem Stich in den Körper eines Patienten eindringt auf den Grundkörper 2 und dort bis zu dem seitlich versetzt angeordneten Testbereich. Bevorzugt ist die Kapillarstruktur 5, wie in Figur 1 dargestellt, zur Spitze hin offen. Möglich ist es aber auch, dass die Kapillarstruktur 4 erst kurz hinter der Spitze in einem Bereich beginnt, der bei einem Stich in den Körper eines Patienten eindringt.

Die Kapillarstruktur 5 verläuft in dem Stechelement 4 und in einem daran anschlie-βenden Bereich des Grundkörpers 2 geradlinig in Stichrichtung. Die Kapillarstruktur 5 hat deshalb einen Knick oder eine Kurve. Durch den gerade in dem Stechelement 4 verlaufenden Anfangsabschnitt der Kapillarstruktur 5 ist eine geometrische Gerade definiert, die in einem Abstand an dem Testbereich 3 vorbei verläuft. Der Testbereich 3 ist nämlich in Bezug auf die Längsrichtung des Stechelements 4 seitlich versetzt von dem Stechelement 4 angeordnet. Auf diese Weise ist in Stichrichtung gesehen seitlich neben dem Testbereich 3 Platz für einen Kopplungsbereich 7 zum Ankoppeln der Lanzette 1 an einen Stechantrieb eines Handgeräts. Der in Figur 1 durch eine durchbrochene Linie angedeutete Kopplungsbereich 7 kann als eine Kopplungsfläche ausgeführt sein, die sich nicht von der übrigen Oberfläche des Grundkörpers 2 unterscheidet, oder mit einer Kopplungsstruktur versehen sein, beispielsweise Rillen oder andere Vertiefungen aufweisen.

Der Kopplungsbereich 7 ist vorteilhaft derart angeordnet ist, dass eine geometrische Gerade, die durch den Anfangsabschnitt der Kapillarstruktur 5 definiert ist, den Kopplungsbereich 7 schneidet. Der Testbereich 3 ist dabei in Bezug auf die Längsrichtung des Stechelements 4 seitlich neben dem Kopplungsbereich 7 angeordnet.

Der Grundkörper 2 der Lanzette 1 ist bevorzugt plattenförmig ausgebildet. Die auf der Oberseite des Grundkörpers 2 ausgebildete Kapillarstruktur 5 ist deshalb bei dem dargestellten Ausführungsbeispiel in einer Ebene angeordnet und bewirkt einen Flüssigkeitstransport in einer Ebene, nämlich auf der im wesentlichen flachen Oberseite des Grundkörpers 2.

Beispielsweise können zur Herstellung der Lanzette 1 die Kontur des Grundkörpers 2 mit dem davon ausgehenden Stechelement 4 aus einem Stahlblech ausgeschnitten werden. Möglich ist es aber auch, den Grundkörper 2 und das Stechelement 4 separat herzustellen und bei der Herstellung der Lanzette 1 miteinander zu verbinden.

Die Figuren 2 und 3 zeigen weitere Ausführungsbeispiele einer Flachlanzette 1. Wie bei dem in Figur 1 dargestellten Ausführungsbeispiel ist das Stechelement 4 in Stichrichtung gesehen seitlich neben dem Testbereich 3 angeordnet. Bei den Ausführungsbeispielen der Figuren 2 und 3 geht das Stechelement 4 jedoch nicht mittig von einer Seite des Grundkörpers 2 aus. Stattdessen ist das Stechelement 4 seitlich versetzt an dem Grundkörper 2 angeordnet.

Bei den in den Figuren 2 und 3 dargestellten Ausführungsbeispielen erstreckt sich der Grundkörper 2 seitlich von dem Stechelement 4 mit seinem den Testbereich 3 tragenden Abschnitt mindestens doppelt so weit wie auf der entgegen gesetzten Seite des Stechelements 4. Bei den Ausführungsbeispielen der Figuren 2 und 3 erstreckt sich der Grundkörper 2 auf der Seite des Stechelements 4, auf welcher der Testbereich 3 angeordnet ist, mehr als doppelt so weit wie auf der anderen Seite des Stechelements 4. Bevorzugt ist dabei, dass der Grundkörper 2 sich auf beiden Seiten seitlich von dem Stechelement 4 erstreckt, wie dies in den Figuren 2 und 3 dargestellt ist. Prinzipiell ist jedoch auch möglich, dass sich der Grundkörper 2 quer zur Stichrichtung nur auf einer Seite des Stechelements 4 erstreckt und auf der anderen Seite bündig mit dem Stechelement 4 endet.

Bevorzugt hat jede Lanzette 1 nur einen einzigen Testbereich 3. Dieser ist bei den dargestellten Ausführungsbeispielen als ein auf eine Oberseite des Grundkörpers aufgeklebtes Testfeld mit Nachweisreagenzien ausgebildet. Die Kapillarstruktur 5 führt bei dem dargestellten Ausführungsbeispiel von dem Stechelement 4 zu dem Testbereich 3 und erstreckt sich über den Testbereich 3 hinaus, so dass der Testbereich 3 eine Körperflüssigkeitsprobe besonders effizient aufnehmen kann. Bevorzugt ist der Testbereich 3, wie in den Figuren dargestellt, streifenförmig ausgebildet.

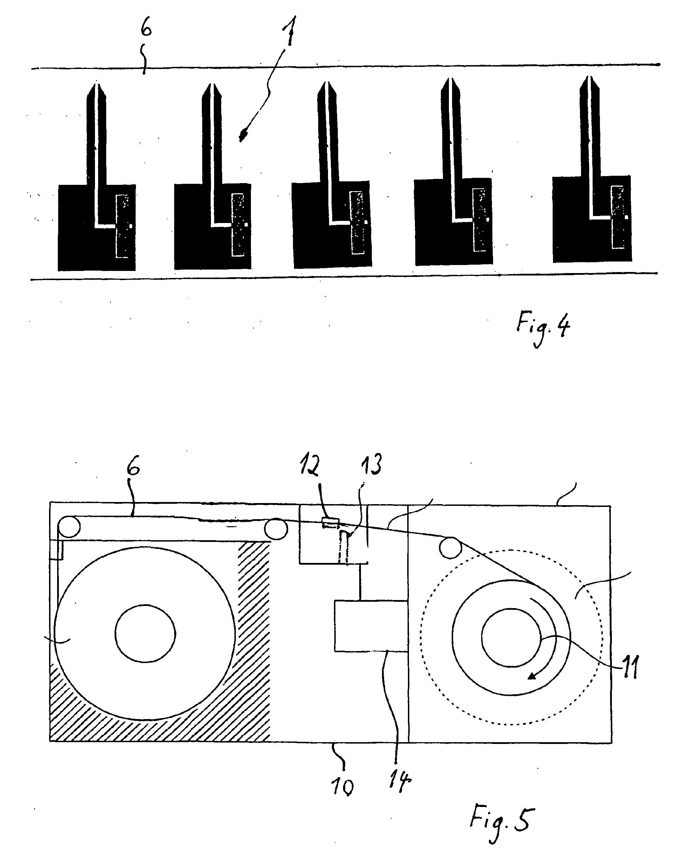

Figur 4 zeigt in einer schematischen Darstellung ein Lanzettenträgerband 6, das mehrere Flachlanzetten 1 trägt, die beispielsweise gemäß Figur 1 ausgebildet sein können. Bei dem dargestellten Ausführungsbeispiel sind die Lanzette 1 quer zur Längsrichtung des Trägerbandes 6, genauer gesagt senkrecht zur Längsrichtung orientiert. Ein solches Lanzettenträgerband 6 kann beispielsweise in einer Bandkassette vertrieben werden, die in ein passendes Aufnahmefach eines Geräts eingesetzt wird.

Figur 5 zeigt eine schematische Darstellung eines Stechsystems, das aus einem Handgerät 10 und Lanzetten 1, wie sie beispielhaft vorstehend beschriebenen wurden, besteht. In dem Stechgerät 10 ist ein Lanzettenträgerband 6 mit Lanzetten 1 angeordnet. Das Handgerät 10 hat eine Wickeleinrichtung 11, um das Trägerband 6 schrittweise aufzuwickeln und so die von dem Trägerband 6 getragenen Lanzetten 1 nacheinander in eine Gebrauchsposition zu bringen, in der sie von einem Stechantrieb 12 in eine geradlinige Stichbewegung versetzt werden können. Der Stechantrieb 12 hat einen Lanzettenhalter, um eine Lanzette 1 zusammen mit einem sie tragenden Trägerbandabschnitt zu klemmen. Bei dem dargestellten Ausführungsbeispiel erfolgt die Stichrichtung senkrecht zur Zeichenebene. Bei einem Stich wird das Lanzettenträgerband 6 umgebogen, so dass sich die Spitze des Stechelements 4 von dem Trägerband 6 abhebt, wie dies aus der WO 2008/083844 A1 bekannt ist.

Das Stechgerät 10 weist ferner eine Messeinheit 13 auf, um eine Körperflüssigkeitsprobe in dem Testbereich 3 zu untersuchen. Da der Testbereich 3 der Lanzetten 1 in Stichrichtung seitlich versetzt hinter dem Stechelement 4 angeordnet ist, lässt der Lanzettenhalter den Testbereich 3 einer gehaltenen Lanzette 1 frei. Der Testbereich 3 und eine von ihm aufgenommene Probe können deshalb von der Messeinheit 13, beispielsweise durch eine photometrische Messung, untersucht werden, während die Lanzette 1 von dem Lanzettenhalter gehalten ist.

Die Messeinheit 13 und der Stechantrieb 12 können mit einer Steuer- und Auswerteeinheit 14 verbunden sein, welche Messungen auswertet und das Anzeigen von Messergebnissen auf einer Anzeigeinrichtung, beispielsweise einer Flüssigkristallanzeige bewirkt.

### Bezugszahlen

- 1: Lanzette
- 2: Grundkörper
- 3: Testbereich
- 4: Stechelement
- 5: Kapillarstruktur
- 6: Lanzettenträgerband
- 7: Kopplungsbereich
- 10: Handgerät
- 11: Wickeleinrichtung
- 12: Stechantrieb
- 13: Messeinheit
- 14: Steuer- und Auswerteeinheit

## Patentansprüche

1. Lanzette mit
einem Grundkörper (2), der auf einer Oberseite einen Testbereich (3) zur Untersuchung einer Körperflüssigkeitsprobe hat,
einem von dem Grundkörper (2) ausgehenden Stechelement (4) zum Einstich in die Haut eines Patienten, und
einer Kapillarstruktur (5), um eine Körperflüssigkeitsprobe von dem Stechelement (4) zu dem Testbereich (3) zu transportieren, wobei zumindest ein Abschnitt der Kapillarstruktur (5) als ein Kapillarkanal ausgebildet ist, und wobei in einer Draufsicht auf die Oberseite gesehen der Testbereich (3) in Bezug auf die Längsrichtung des Stechelements (4) seitlich versetzt von dem Stechelement (4) angeordnet ist und die Kapillarstruktur (5) einen Abschnitt aufweist, der Flüssigkeit seitwärts transportiert, und wobei
der Grundkörper (2) einen Kopplungsbereich (7) zum Ankoppeln der Lanzette (1) an einen Stechantrieb (12) eines Handgeräts (10) aufweist,
in dem Stechelement (4) ein gerade verlaufender Anfangsabschnitt der Kapillarstruktur (5) angeordnet ist, und
der Koppjungsbereich (7) derart angeordnet ist, dass eine geometrische Gerade, die durch den Anfangsabschnitt der Kapillarstruktur (5) definiert ist, den Kopplungsbereich (7) schneidet,
**dadurch gekennzeichnet, dass** ,
der Kapillarkanal (5) einen Knick mit angerundeten Ecken oder eine Kurve aufweist und im Bereich des Knicks oder der Kurve eine Querschnittsfläche aufweist, die höchstens so groß wie in einem angrenzenden Abschnitt ist, und wobei der Kopplungsbereich (7) in gerader Linie hinter dem Stechelement (4) und seitlich neben dem Testbereich (3) angeordnet ist.

2. Lanzette nach Anspruch 1, **dadurch gekennzeichnet, dass** der Testbereich (3) in Bezug auf die Längsrichtung des Stechelements (4) seitlich mindestens um die Breite des Stechelements (4) versetzt angeordnet ist.

3. Lanzette nach einem der vorstehenden Ansprüche, **dadurch gekennzeichnet, dass** der Grundkörper (2) plattenförmig ist.

4. Lanzette nach einem der vorstehenden Ansprüche, **dadurch gekennzeichnet, dass** die Kapillarstruktur (5) einen Flüssigkeitstransport in einer Ebene bewirkt.

5. Lanzette nach einem der vorstehenden Ansprüche, **dadurch gekennzeichnet, dass** der Grundköper (2) einstückig mit dem Stechelement (4) ausgebildet ist.

6. Lanzette nach einem der vorstehenden Ansprüche, **dadurch gekennzeichnet, dass** der Testbereich (3) derart angeordnet ist, dass eine geometrische Gerade, die durch den Anfangsabschnitt der Kapillarstruktur (5) definiert ist, in einem Abstand an dem Testbereich (3) vorbei verläuft.

7. Lanzette nach einem der vorstehenden Ansprüche, dadurch gekenntzeichnet dass das Stechelement (4) in einer Draufsicht außermittig an dem Grundkörper (2) angeordnet ist.

8. Lanzette nach einem der vorstehenden Ansprüche, **dadurch gekennzeichnet, dass** die Lanzette (1) nur einen einzigen Testbereich (3) aufweist.

9. Lanzettenträgerband mit mehreren Lanzetten (1) nach einem der vorstehenden Ansprüche.

10. Stechsystem mit
Lanzetten (1) nach einem der vorstehenden Ansprüche und
einem Stechgerät (10), das einen Stechantrieb (12) aufweist, um die Lanzetten (1) in eine geradlinige Stichbewegung zu versetzen, und eine Messeinheit (13) aufweist, um eine Körperflüssigkeitsprobe in dem Testbereich (3) zu untersuchen.

## Claims

1. A lancet, comprising
a base body (2), which has a test region (3) on an upper face for analyzing a body fluid sample,
a lancing element (4) extending from the base body (2) for puncturing the skin of a patient, and
a capillary structure (5) for transporting a body fluid sample from the lancing element (4) to the test region (3), wherein at least a section of the capillary structure (5) is a capillary channel, and wherein
in a top view of the upper face, the test region (3) is disposed laterally offset in relation to the longitudinal direction of the lancing element (4), and the capillary structure (5) has a section that transports fluid sideways, and wherein
the base body (2) comprises a coupling region (7) for coupling the lancet (1) to a lancing drive (12) of a hand-held device (10),
a rectilinear starting section of the capillary structure (5) is arranged in the lancing element (4), and
the coupling region (7) is disposed so that a geometrical straight line, which is defined by the starting section of the capillary structure (5), intersects the coupling region (7),
**characterized in that**
the capillary channel (5) has a bend with rounded corners or a curve and a cross-sectional surface, in the region of the bend or curve, that is no larger than in an adjoining section, and wherein
the coupling region (7) is disposed in a straight line behind the lancing element (4) and laterally beside the test region (3).

2. The lancet according to claim 1, **characterized in that** the test region (3) is disposed laterally offset by at least the width of the lancing element (4) with respect to the longitudinal direction of the lancing element (4).

3. A lancet according to any one of the preceding claims, **characterized in that** the base body (2) is plate-shaped.

4. A lancet according to any one of the preceding claims, **characterized in that** the capillary structure (5) causes fluid to be transported in a plane.

5. A lancet according to any one of the preceding claims, **characterized in that** the base body (2) is integral with the lancing element (4).

6. The lancet according to any one of the preceding claims, **characterized in that** the test region (3) is disposed such that a geometrical straight line, which is defined by the starting section of the capillary structure (5), passes the test region (3) at a distance.

7. A lancet according to any one of the preceding claims, **characterized in that** the lancing element (4) is disposed eccentrically on the base body (2) as viewed from above.

8. A lancet according to any one of the preceding claims, **characterized in that** the lancet (1) comprises only a single test region (3).

9. A lancet carrier tape, comprising a plurality of lancets (1) according to any one of the preceding claims.

10. A lancing system, comprising
lancets (1) according to any one of the preceding claims, and
a lancing device (10), which comprises a lancing drive (12) so as to cause the lancets (1) to perform a rectilinear piercing movement, and a measuring unit (13) for analyzing a body fluid sample in the test region (3).

## Revendications

1. Lancette comprenant
un corps de base (2) qui a sur un côté supérieur une zone de test (3) destinée à l'analyse d'un échantillon de liquide biologique,
un élément de piqûre (4) partant du corps de base (2), destiné à piquer dans la peau d'un patient, et
une structure capillaire (5) afin de transporter un échantillon de liquide biologique de l'élément de piqûre (4) vers la zone de test (3), au moins un segment de la structure capillaire (5) étant réalisé comme canal capillaire, et,
la zone de test (3), en vue de dessus sur le côté supérieur, étant disposée de manière latéralement décalée par rapport au sens longitudinal de l'élément de piqûre (4) et la structure capillaire (5) présentant un segment qui transporte le liquide latéralement, et
le corps de base (2) présentant une zone de couplage (7) destinée à coupler la lancette (1) à un entraînement de piqûre (12) d'un appareil manuel (10),
un segment de départ de la structure capillaire (5) s'étendant de manière rectiligne étant disposé dans l'élément de piqûre (4), et
la zone de couplage (7) étant disposée de manière à ce qu'une droite géométrique, définie par le segment de départ de la structure capillaire (5), coupe la zone de couplage (7),
**caractérisée en ce que**
le canal capillaire (5) présente un coude à coins arrondis ou une courbe et, dans la partie du coude ou de la courbe, présente une surface de la section transversale qui est au maximum aussi grande que dans un segment adjacent, et
la zone de couplage (7) étant disposée en ligne droite derrière l'élément de piqûre (4) et latéralement à côté de la zone de test (3).

2. Lancette selon la revendication 1, **caractérisée en ce que** la zone de test (3) est disposée latéralement de manière décalée d'au moins la largeur de l'élément de piqûre (4) par rapport au sens longitudinal de l'élément de piqûre (4).

3. Lancette selon l'une quelconque des revendications précédentes, **caractérisée en ce que** le corps de base (2) est en forme de plaque.

4. Lancette selon l'une quelconque des revendications précédentes, **caractérisée en ce que** la structure capillaire (5) produit un transport de liquide dans un plan.

5. Lancette selon l'une quelconque des revendications précédentes, **caractérisée en ce que** le corps de base (2) est réalisé d'une seule pièce avec l'élément de piqûre (4).

6. Lancette selon l'une quelconque des revendications précédentes, **caractérisée en ce que** la zone de test (3) est disposée de manière à ce qu'une droite géométrique, définie par le segment de départ de la structure capillaire (5), passe devant la zone de test (3) avec écart.

7. Lancette selon l'une quelconque des revendications précédentes, **caractérisée en ce que** l'élément de piqûre (4), en vue de dessus, est disposé excentriquement sur le corps de base (2).

8. Lancette selon l'une quelconque des revendications précédentes, **caractérisée en ce que** la lancette (1) ne présente qu'une seule zone de test (1).

9. Bande-support de lancettes comprenant plusieurs lancettes selon l'une quelconque des revendications précédentes.

10. Système de piqûre comprenant
des lancettes (1) selon l'une quelconque des revendications précédentes et
un appareil de piqûre (10) qui présente un entraînement de piqûre (12) afin de mettre les lancettes (1) dans un mouvement de piqûre rectiligne, et une unité de mesure (13) afin d'analyser un échantillon de liquide biologique dans la zone de test (3).
